# EUROPEAN PATENT SPECIFICATION

(11) **EP 4 062 973 B1**
(45) Date of publication and mention of the grant of the patent: **16.09.2026**
(21) Application number: 20890315.3
(22) Date of filing: 18.11.2020
(51) Int. Cl.: A61P 25/04, A61P 43/00, A61K 9/10, A61K 9/14, A61K 9/16, A61K 47/02, A61K 47/12, A61K 47/20, A61K 47/22, A61K 31/485

(54) **6,7-UNSATURATED-7-CARBAMOYL MORPHINAN DERIVATIVE-CONTAINING SOLID FORMULATION**
6,7-UNGESÄTTIGTES-7-CARBAMOYL-MORPHINANDERIVAT ENTHALTENDE FESTE FORMULIERUNG
FORMULATION SOLIDE CONTENANT UN DÉRIVÉ DE MORPHINANE 6,7-INSATURÉ DU 7-CARBAMOYL

(30) Priority: 20.11.2019 JP 2019209630
(43) Date of publication of application: 28.09.2022
(73) Proprietor: Shionogi & Co., Ltd., Osaka-shi, Osaka 541-0045 (JP)
(72) Inventor: OHASHI, Ryo, Amagasaki-shi, Hyogo 660-0813 (JP); TOKUDA, Taketo, Amagasaki-shi, Hyogo 660-0813 (JP); OSAKA, Takeshi, Amagasaki-shi, Hyogo 660-0813 (JP)
(74) Representative: Vossius & Partner Patentanwälte Rechtsanwälte mbB
(86) International application number: PCT/JP2020/042889
(87) International publication number: WO 2021/100728

(56) References cited:
- WO-A1-2012/063933
- WO-A1-2013/172297
- WO-A1-2013/172297
- WO-A1-2018/021518
- WO-A1-2018/124062
- WO-A1-99/02158
- WO-A1-99/02158
- WO-A2-03/077867

## Description

### [TECHNICAL FIELD]

The present invention relates to a solid preparation containing 6,7-unsaturated-7-carbamoyl morphinan derivatives, especially, a solid preparation which improves stability of 17-(cyclopropylmethyl)-6,7-didehydro-4,5a-epoxy-3,6,14-trihydroxy-N-[2-(3-phenyl-1,2,4-oxadiazol-5-yl)propan-2-yllmorphinan-7-carboxamide 4-methylbenzenesulfonic acid.

### [BACKGROUND ART]

An agonist on opioid receptor, such as morphine, is used as very effective analgesic for patients suffering from cancerous pain, but it induces the strong nausea, retching, constipation, ischuria, itch and the like as side effects. The various antiemetic and anti-constipation are used clinically. However, all of them do not exhibit a sufficient effect, and an excellent agent for reducing an adverse effect is requested to improve QOL of patients.

Patent Document 1 discloses a 6,7-unsaturated-7-carbamoyl morphinan derivative (hereinafter, may be referred to as "the present derivative") as the agent for reducing an adverse effect for the opioid receptor agonist. Among the present derivatives, especially, 17-(cyclopropylmethyl)-6,7-didehydro-4,5a-epoxy-3,6,14-trihydroxy-N-[2-(3-phenyl-1,2,4-oxadiazol-5-yl)propan-2-yl]morphinan-7-carboxamide 4-methyl benzenesulfonic acid (Compound A, hereinafter, may be referred to as "the present compound"), which is effective as the above agent for reducing an adverse effect, is effective. Patent Document 2 discloses a solid preparation containing the present compound.

A child may take the present compound. When a child takes medicine orally, bitterness may be a problem. In this case, a syrup having sweetness may be taken. In General Rules for Preparations of the Japanese Pharmacopoeia, syrups are defined as "viscous liquid or solid preparations containing sugars or sweetening agents, intended for oral administration." Syrups include dry syrups as "preparations to be used after having been dissolved or suspended". The dry syrup is solid, and can be taken after having been dissolved or suspended in water, and thus has higher storage stability and better portability than liquid. However, an active ingredient in the dry syrup may be decomposed after storage over time, and particularly, when the dry syrup is dissolved or suspended in water, the active ingredient may be decomposed.

When the active ingredient in the preparation is decomposed, an additive agent may be blended to suppress decomposition. Cited Documents 1 and 2 do not disclose suppressing the decomposition of the present derivative or the present compound in water. On the other hand, Cited Documents 3 to 6 disclose a preparation in which stability of a compound is improved by blending an additive agent. However, the chemical structure between the compounds in the above documents and 6,7-unsaturated-7-carbamoyl morphinan derivatives is very different, and it has not been disclosed that stability, especially, stability in water, is improved when the additive agent is blended.
Patent Document 7 relates to a p-toluenesulfonic acid salt, an acetic acid salt or a hydrochloric acid salt of specific 6,7-unsaturated-7-carbamoyl morphinan derivatives or a solvate of the compound or an acid addition salt thereof.
Patent Document 8 concerns a pharmaceutical composition comprising, a 4,5-epoxymorphinan derivative and at least one substance selected from the group consisting of the following materials (1), (2), (3), (4) and (5):
(1) a water soluble antioxidant selected from the group consisting of sodium sulfite, sodium hydrogensulfite, sodium pyrosulfite, Rongalite, sodium nitrite, L-ascorbic acid, erysorbic acid, sodium thiosulfate, sodium thiomalate, cysteine, thioglycerol, and hydroxyquinoline sulfate;
(2) a fat soluble antioxidant selected from the group consisting of propyl gallate, butyl hydroxytoluene, butyl hydroxyanisole, tocopherol, ascorbyl palmitate, ascorbyl stearate, nordihydroguaiaretic acid, and mercaptobenzimidazole;
(3) a synergist selected from the group consisting of EDTA, salts thereof, citric acid, salts thereof, and lecithin;
(4) a sugar selected from the group consisting of D-mannitol, D-sorbitol, xylitol, glucose, and fructose; and
(5) a surfactant selected from the group consisting of sorbitan sesquioleate, sorbitan laurate, sorbitan palmitate, glyceryl myristate, polyoxyethylene nonylphenyl ether, and polyoxyethylene lauryl ether.
Patent Document 9 relates to a solid preparation comprising an active ingredient composed of a specific 4,5-epoxymorphinan derivative or a pharmaceutically acceptable acid addition salt thereof, and one or more stabilizing agents selected from the group consisting of n-propyl gallate, sodium hydrogensulfite, dibutylhydroxytoluene, butylhydroxyanisole, tocopherol and D-isoascorbic acid, wherein the amount of the active ingredient is 0.00001 to 0.01% by weight of the solid preparation, and wherein the amount of the stabilizing agent is 0.005 to 5% by weight of the solid preparation.

### [PRIOR ART REFERENCES]

### [Patent Document]

[Patent Document 1] WO 2006/126637 A
[Patent Document 2] WO 2013/172297 A
[Patent Document 3] JP 2009-143948 A
[Patent Document 4] JP 11-92369 A
[Patent Document 5] JP 2014-9226 A
[Patent Document 6] JP 2013-514386 A
[Patent Document 7] WO 2012/063933 A1
[Patent Document 8] WO 99/02158 A1
[Patent Document 9] WO 2018/021518 A1

### [SUMMARY OF THE INVENTION]

### [PROBLEMS TO BE SOLVED BY THE INVENTION]

Therefore, development of a preparation with improved stability of the present derivative and the present compound, especially, stability in water of the present derivative and the present compound, and improved temporal stability has been demanded.

### [MEANS FOR SOLVING THE PROBLEM]

The present inventors intensively studied, and found out that stability can be improved by optimum additive agents in a preparation containing the present derivative or the present compound, thus completed the present invention. That is, when one or more selected from the group consisting of ascorbic acid or its derivative, sulfur-containing amino acid or its derivative, sulfite or its derivative, citric acid or its derivative and malic acid or its derivative are used as the stabilizer, stability, especially, stability in water, can be increased.

That is, the present invention is as defined in the claims.

### [EFFECT OF THE INVENTION]

When the preparation containing 6,7-unsaturated-7-carbamoyl morphinan derivatives of the present invention contains one or more selected from the group consisting of ascorbic acid or its derivative, sulfur-containing amino acid or its derivative, sulfite or its derivative, citric acid or its derivative and malic acid or its derivative in the preparation, the amount of related substances, especially, the amount of related substances after suspending the present preparation in water, can be reduced.

### [MODE FOR CARRYING OUT THE INVENTION]

The agent for reducing an adverse effect for the opioid receptor agonist as an active ingredient used in the present invention is usually a 6,7-unsaturated-7-carbamoyl morphinan derivative, preferably the compound represented by formula (IA) (Compound A) or its pharmaceutically acceptable salt. It is more preferably a pharmaceutically acceptable salt of the compound represented by formula (IA).

Examples of the pharmaceutically acceptable salt of the compound represented by formula (IA) include salts of the compound represented by formula (IA) with alkali metals (for example lithium, sodium, potassium and the like), alkaline earth metals (for example calcium, barium and the like), magnesium, transition metals (for example zinc, iron and the like), ammonia, organic bases (for example trimethylamine, triethylamine, dicyclohexylamine, ethanolamine, diethanolamine, triethanolamine, meglumine, diethanolamine, ethylenediamine, pyridine, picoline, quinolone and the like), and amino acid, or salts with inorganic acids (for example hydrochloric acid, sulfuric acid, nitric acid, carbonic acid, hydrobromic acid, phosphoric acid and hydroiodic acid and the like) and organic acids (for example formic acid, acetic acid, propionic acid, trifluoroacetic acid, citric acid, lactic acid, tartaric acid, oxalic acid, maleic acid, fumaric acid, mandelic acid, glutaric acid, malic acid, benzoic acid, phthalic acid, ascorbic acid, benzenesulfonic acid, p-toluenesulfonic acid, methanesulfonic acid, ethanesulfonic acid and the like). Particularly, examples of salts include salts with hydrochloric acid, sulfuric acid, phosphoric acid, tartaric acid, or methanesulfonic acid. Preferable is salts with acid. These salts can be formed by a conventional method.

The salt is particularly preferably p-toluenesulfonic acid salt, acetate or hydrochloride salt of the compound represented by formula (IA), and further preferably a crystal of p-toluenesulfonic acid salt of the compound represented by formula (IA), or a crystal of a solvate of the salt. The salt is particularly preferably 17-(cyclopropylmethyl)-6,7-didehydro-4,5α-epoxy-3,6,14-trihydroxy-N-[2-(3-phenyl-1,2,4-oxadiazol-5-yl)propan-2-yl]morphinan-7-carboxamide 4-methylbenzenesulfonic acid.

The crystal of p-toluenesulfonic acid of the compound represented by formula (IA) (Compound A) does not have the hygroscopicity and have the excellent stability. In addition, the crystal exists in a state of crystal in the solid preparation. The present compound, the production method of the crystal of the compound and the like are described in claims and descriptions in International Publication WO 2006/126637A and International Publication WO2012/063933A.

As the active ingredient in the preparation of the present invention, a compound represented by formula (IA): 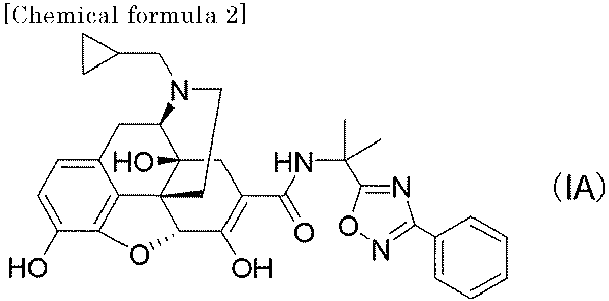 or its pharmaceutically acceptable salt is used. The active ingredient is preferably a p-toluenesulfonic acid salt, acetate or hydrochloride salt of the compound represented by formula (IA), and more preferably a p-toluenesulfonic acid salt of the compound represented by formula (IA).

Methods for producing the compound represented by formula (IA), its pharmaceutically acceptable salt, and the p-toluenesulfonic acid salt of the compound represented by formula (IA) are disclosed in International Publication WO 2006/126637A and International Publication WO 2012/063933A.

Examples of the p-toluenesulfonic acid salt of the compound represented by formula (IA) that is the present compound include 17-(cyclopropylmethyl)-6,7-didehydro-4,5α-epoxy-3,6,14-trihydroxy-N-[2-(3-phenyl-1,2,4-oxadiazol-5-yl)propan-2-yl]morphinan-7-carboxamide 4-methylbenzenesulfonic acid.

A content of the compound represented by formula (IA) that is the present derivative in the preparation of the present invention, the p-toluenesulfonic acid salt, acetate or hydrochloride salt of the compound represented by formula (IA), especially a content of the present compound, is not particularly limited, and may be an amount at which drug efficacy is obtained. For example, the content is 0.001 to 10% by weight, preferably 0.001 to 5% by weight, more preferably 0.001 to 2.5% by weight, and particularly preferably 0.001 to 2% by weight, based on the total amount of the preparation. When the content is more than these contained amounts, there is a possibility that a dose is increased, and when the content is less than these contained amounts, there is a possibility that a decomposed amount of active ingredient is greater.

In order to stabilize the present derivative, the present compound, and the p-toluenesulfonic acid salt, acetate or hydrochloride salt of the compound represented by formula (IA) in the preparation of the present invention, one or more stabilizers selected from the group consisting of ascorbic acid, sodium L-ascorbate, erythorbic acid, L-ascorbic acid stearic acid ester, L-ascorbic acid palmitic acid ester, L-cysteine, L-cysteine hydrochloride, L-cysteine hydrochloride hydrate, L-methionine, DL-methionine, sodium pyrosulfite, sodium sulfite, anhydrous citric acid, citric acid hydrate, calcium citrate, sodium citrate hydrate, sodium dihydrogen citrate, disodium citrate, DL-malic acid and sodium DL-malate is/are contained in the preparation of the present invention. Further stabilizers may be any stabilizer as long as it can stabilize the present derivative and the present compound, and is preferably listed in the Japanese Pharmacopoeia, the Japanese Pharmaceutical Codex, the Japanese Pharmaceutical Excipients, the Japanese Standards of Food Additives, the United States Pharmacopoeia (USP), and the European Pharmacopoeia (Ph.Eur.), and more preferably used as an antioxidant. As used herein, the term "ascorbic acid or its derivative" refers to ascorbic acid, sodium L-ascorbate, erythorbic acid which is a stereoisomer of ascorbic acid, L-ascorbic acid stearic acid ester, and L-ascorbic acid palmitic acid ester; the term "sulfur-containing amino acid or its derivative" refers to L-cysteine, L-cysteine hydrochloride, L-cysteine hydrochloride hydrate, L-methionine, and DL-methionine; the term "sulfite or its derivative" refers to sodium pyrosulfite and sodium sulfite; the term "citric acid or its derivative" refers to anhydrous citric acid, citric acid hydrate, calcium citrate, sodium citrate hydrate, sodium dihydrogen citrate, and disodium citrate; the term "malic acid or its derivative" refers to DL-malic acid and sodium DL-malate; and the term "edetic acid or its derivative" refers to sodium edetate, sodium edetate hydrate, calcium disodium edetate, tetrasodium edetate, and tetrasodium edetate tetrahydrate.
Preferred examples thereof include "ascorbic acid or its derivative" such as ascorbic acid, sodium L-ascorbate, and erythorbic acid which is a stereoisomer of ascorbic acid; "sulfur-containing amino acid or its derivative" such as L-cysteine hydrochloride hydrate and L-methionine; "sulfite or its derivative" such as sodium pyrosulfite and sodium sulfite; "citric acid or its derivative" such as anhydrous citric acid; and "malic acid or its derivative" such as DL-malic acid. More preferred are "ascorbic acid or its derivative" such as ascorbic acid, sodium L-ascorbate, and erythorbic acid which is a stereoisomer of ascorbic acid; and "sulfur-containing amino acid or its derivative" such as L-cysteine hydrochloride hydrate and L-methionine. Further preferred are ascorbic acid and L-methionine, and particularly preferred is ascorbic acid. As set out in claim 1, the solid preparation of the present invention comprises one or more stabilizers selected from the group consisting of ascorbic acid, sodium L-ascorbate, erythorbic acid, L-ascorbic acid stearic acid ester, L-ascorbic acid palmitic acid ester, L-cysteine, L-cysteine hydrochloride, L-cysteine hydrochloride hydrate, L-methionine, DL-methionine, sodium pyrosulfite, sodium sulfite, anhydrous citric acid, citric acid hydrate, calcium citrate, sodium citrate hydrate, sodium dihydrogen citrate, disodium citrate, DL-malic acid and sodium DL-malate. These stabilizers may be used singly or in combination of two or more kinds thereof. Moreover, the additive agents described above may be used for other purposes in addition to being used as a stabilizer.

The content of the stabilizer of the preparation of the present invention is not particularly limited, and may be any amount as long as the amount of related substances of the present compound is small and the present compound can be stabilized. For example, the content of the stabilizer is 1 to 2500 parts by weight, preferably 2 to 1250 parts by weight, more preferably 3 to 1000 parts by weight, based on 1 part by weight of the present compound, especially the compound represented by formula (IA), the p-toluenesulfonic acid salt, acetate or hydrochloride salt of the compound represented by formula (IA). The content of the stabilizer is usually 0.005 to 30% by weight, preferably 0.01 to 25% by weight, more preferably 0.1 to 20% by weight, based on the total amount of the preparation. When the content is more than these contents, there is a possibility that the dose is increased, and when the content is less than these contents, there is a possibility that the active ingredient in the preparation is not stabilized.

The related substances of the present compound in the preparation of the present invention is affected highly by the stabilizer. Examples of a main related substances of the present compound include a hydroxide form of the compound represented by formula (IA) and a carboxylic acid form of the compound represented by formula (IA).

Regarding the amount of related substances of the present compound in the preparation of the present invention, in the initial, the total of all related substances (hereinafter, may be referred to as "total related substances") of the compound represented by formula (IA) is 4.0% or less. Here, the initial means before the start of the temporal stability test. When the amount is more than this amount of related substances, there is a possibility that toxicity occurs.

When the preparation of the present invention is suspended in water at room temperature for 3 hours, the total amount of related substances of the compound represented by formula (IA) is 4.0% or less. When the amount is more than this amount of related substances, there is a possibility that toxicity occurs.

When the preparation of the present invention is suspended in water at room temperature for 3 hours, the amount of the hydroxide form of the compound represented by formula (IA) or the carboxylic acid form of the compound represented by formula (IA) is 1.0% or less. This amount satisfies the safety confirmation thresholds of ICH (International Conference on Harmonization) Q3B guidelines. When the amount is more than this amount of related substances, there is a possibility that toxicity occurs.

Even when the preparation of the present invention is stored at 40°C and a relative humidity of 75% for 1 week, the total amount of related substances of the compound represented by formula (IA) is 4.0% or less, and the amount of the hydroxide form of the compound represented by formula (IA) or the carboxylic acid form of the compound represented by formula (IA) is 1.0% or less.

The preparation of the present invention may contain an excipient, and excipients listed in the Japanese Pharmacopoeia, the Japanese Pharmaceutical Codex, the Japanese Pharmaceutical Excipients, the Japanese Standards of Food Additives, the United States Pharmacopoeia (USP), and the European Pharmacopoeia (Ph.Eur.) can be used. Examples of the excipient include sugar derivatives, starch derivatives, cellulose derivatives, inorganic excipients, β-cyclodextrin, magnesium stearate, crospovidone, calcium stearate, sucrose fatty acid ester, powdered tragacanth, gum arabic, dextran, and pullulan.

Examples of the sugar derivatives include sugars and sugar alcohols, examples of the sugars include lactose, white soft sugar, glucose, fructose, sucrose, and maltose, and examples of the sugar alcohols include D-mannitol, D-sorbitol, erythritol, xylitol, powdered maltose starch syrup, maltitol, and trehalose. In addition, examples of other sugar derivatives include fructooligosaccharides, palatinose, maltose, reduced maltose, powdered sugar, starch syrup, lactulose, reduced lactose lactitol, and honey sugar.

Examples of the starch derivatives include starch, potato starch, corn starch (cornstarch), rice starch, partly pregelatinized starch, pregelatinized starch, porous starch, wheat starch, carboxystarch sodium, hydroxypropyl starch, low-substituted carboxymethyl starch, and carboxymethyl starch.

Examples of the cellulose derivatives include crystalline cellulose, powder cellulose, carmellose sodium, carmellose, croscarmellose sodium, carmellose calcium, carboxymethylethylcellulose, and low-substituted hydroxypropylcellulose.

Examples of the inorganic excipients include silicate derivatives, phosphates, carbonates, sulfates, magnesium oxide, titanium oxide, calcium lactate, synthetic hydrotalcite, talc, kaolin, dried aluminum hydroxide, magnesium oxide, and bentonite.

Examples of the silicate derivatives include silicon dioxides such as hydrated silicon dioxide and light anhydrous silicic acid, magnesium aluminometasilicate, synthetic aluminum silicate, and calcium silicate.

Examples of the phosphates include anhydrous dibasic calcium phosphate, anhydrous calcium phosphate, calcium monohydrogen phosphate, calcium hydrogen phosphate, sodium hydrogen phosphate, dipotassium phosphate, potassium dihydrogen phosphate, calcium dihydrogen phosphate, monobasic sodium phosphate, and silicic anhydride.

Examples of the carbonates include precipitated calcium carbonate, calcium carbonate, and magnesium carbonate. Examples of the sulfates include calcium sulfate.

The excipient of the preparation of the present invention preferably includes D-mannitol.

These excipients may be used singly or in combination of two or more kinds thereof. Moreover, the additive agents described above may be used for other purposes in addition to being used as an excipient.

The particle size of the excipient of the preparation of the present invention may be any particle size at which the preparation can be produced. As for the particle size of the excipient, the volume average particle diameter is 1 to 1000 µm, preferably 10 to 750 µm, more preferably 50 to 500 µm, and particularly preferably 75 to 250 µm. When the particle size is larger than this, there is a possibility that it is difficult to produce a preparation as a granule or a powder, and when the particle size is smaller, there is a possibility that the powder is greatly scattered at the time of production, and the content of the active ingredient is also affected.

A content of the excipient of the preparation of the present invention is not particularly limited, and is usually 50 to 99.9% by weight, preferably 60 to 99.5% by weight, more preferably 70 to 99.0% by weight, based on the total amount of the preparation. When the content is less than these contents, there is a possibility that it is difficult to form a preparation. When two or more kinds of excipients are used, the total amount of the excipients may be within the above content ranges.

The preparation of the present invention may contain a disintegrant, and disintegrants listed in the Japanese Pharmacopoeia, the Japanese Pharmaceutical Codex, the Japanese Pharmaceutical Excipients, the Japanese Standards of Food Additives, the United States Pharmacopoeia (USP), and the European Pharmacopoeia (Ph.Eur.) can be used. Examples of the disintegrant include cellulose type disintegrants, starch type disintegrants, vinyl type disintegrants, and magnesium aluminometasilicate. Examples of the cellulose type disintegrant include croscarmellose sodium (Ac-Di-Sol), carmellose sodium, carmellose calcium, carmellose, crystalline cellulose, crystalline cellulose - carmellose sodium, low-substituted hydroxypropylcellulose, hydroxyethylmethylcellulose, hydroxypropylcellulose, hydroxypropylmethylcellulose, and powder cellulose. Examples of the starch type disintegrants include sodium carboxymethyl starch, hydroxypropyl starch, corn starch, wheat starch, potato starch, pregelatinized starch, partially pregelatinized starch, cornstarch, low-substituted sodium carboxymethyl starch, sodium starch glycolate, and starch. Examples of the vinyl type disintegrants include polyvinylpyrrolidone, crospovidone, and polyvinyl alcohol. The disintegrant is preferably cellulose type disintegrant or vinyl type disintegrant, and in the case of cellulose type disintegrant, more preferably a sodium salt such as croscarmellose sodium, carmellose sodium, crystalline cellulose - carmellose sodium, or sodium carboxymethyl starch, further preferably croscarmellose sodium or crospovidone, particularly preferably croscarmellose sodium. These disintegrants may be used singly or in combination of two or more kinds thereof. Moreover, the additive agents described above may be used for other purposes in addition to being used as a disintegrant.

A content of the disintegrant in the preparation of the present invention is not particularly limited, and is usually 0.01 to 10.0% by weight, preferably 0.05 to 7.5% by weight, more preferably 0.1 to 5.0% by weight, based on the total amount of the preparation. In addition, the content of the disintegrant is 1 to 100 parts by weight, preferably 5 to 80 parts by weight, more preferably 10 to 60 parts by weight, based on 1 part by weight of the compound used in the preparation of the present invention. When the content is more than these contents, there is a possibility that the dose is increased, and when the content is less than these contents, there is a possibility that disintegration and dissolution of the preparation cannot be sufficiently obtained. When two or more disintegrants are used, the total amount of the disintegrants may be within the above content ranges.

The preparation of the present invention may contain a suspending agent that suspends a drug in water, and agents listed in the Japanese Pharmacopoeia, the Japanese Pharmaceutical Codex, the Japanese Pharmaceutical Excipients, the Japanese Standards of Food Additives, the United States Pharmacopoeia (USP), and the European Pharmacopoeia (Ph.Eur.) can be used. The suspending agent may also be referred to as a suspension. Examples of the suspending agent include gum arabic, sodium alginate, kaolin, carrageenan, carboxyvinyl polymer, carmellose, carmellose calcium, carmellose sodium, xanthan gum, glycerin fatty acid ester, light anhydrous silicic acid, crystalline cellulose, crystalline cellulose ·carmellose sodium, sucrose fatty acid ester, sorbitan fatty acid ester, hypromellose, hydroxypropylcellulose, propylene glycol, propylene glycol fatty acid ester, polyvinylpyrrolidone (povidone), polyoxyethylene hydrogenated castor oil, polysorbate 20, polysorbate 60, polysorbate 80, Macrogol 4000, Macrogol 6000, methylcellulose, ethylene glycol monolaurate, sorbitan monolaurate, and polyoxyethylene sorbitol monolaurate. The suspending agent is preferably hypromellose, hydroxypropylcellulose or methylcellulose, more preferably hypromellose or hydroxypropylcellulose, and particularly preferably hypromellose. These suspending agents may be used singly or in combination of two or more kinds thereof. Moreover, the additive agents described above may be used for other purposes in addition to being used as a suspending agent.

A content of the suspending agent in the preparation of the present invention is not particularly limited, and is usually 0.01 to 10.0% by weight, preferably 0.05 to 5.0% by weight, more preferably 0.1 to 2.5% by weight, based on the total amount of the preparation. In addition, the content of the disintegrant is 1 to 100 parts by weight, preferably 2.5 to 50 parts by weight, more preferably 5 to 25 parts by weight, based on 1 part by weight of the compound used in the preparation of the present invention. When the content is more than these contents, there is a possibility that the dose is increased, and when the content is less than these contents, there is a possibility that the drug is not sufficiently suspended in water. When two or more kinds of suspending agents are used, the total amount of the suspending agents may be within the above content ranges.

The preparation of the present invention may contain a binder, and binders listed in the Japanese Pharmacopoeia, the Japanese Pharmaceutical Codex, the Japanese Pharmaceutical Excipients, the Japanese Standards of Food Additives, the United States Pharmacopoeia (USP), and the European Pharmacopoeia (Ph.Eur.) can be used. Examples of the binder include cellulose type binders, starch type binders, vinyl type binders, polyols, gum arabic, gum arabic powder, powdered gum arabic, alginic acid, sodium alginate, sucrose, gelatin, dextrin, pullulan, tragacanth, powdered tragacanth, xanthane gum, pectin, sodium polyacrylate, agar, powdered phellodendron bark, guar gum, light anhydrous silicic acid, and hardened oil.

Examples of the cellulose type binders include carboxymethylcellulose (carmellose, CMC), carboxymethylcellulose sodium (carmellose sodium), hydroxyethyl cellulose (HEC), hydroxypropylcellulose (HPC), hypromellose (hydroxypropyl methylcellulose) (HPMC), methylcellulose (MC), crystalline cellulose, microcrystalline cellulose, ethylcellulose, crystalline cellulose ·carmellose sodium, carmellose calcium, powder cellulose, and low-substituted hydroxypropylcellulose.

Examples of the starch type binders include starch, pregelatinized starch, partly pregelatinized starch, potato starch, wheat starch, rice starch, porous starch, corn starch, hydroxypropyl starch, and sodium starch glycolate (sodium carboxymethyl starch).

Examples of the vinyl type binders include polyvinyl alcohol (PVA), polyvinylpyrrolidone (povidone) (PVP), carboxy vinyl polymer, and copolyvidone (subordinate concept).

Examples of the polyols include Macrogol (polyethylene glycol) 200, Macrogol 300, Macrogol 400, Macrogol 600, Macrogol 1000, Macrogol 1500, Macrogol 1540, Macrogol 4000, Macrogol 6000, Macrogol 20000, glycerin, polyoxyethylene [105] polyoxypropylene [5] glycol, and propylene glycol.

The binder is preferably a cellulose type binder, more preferably sodium carboxymethylcellulose (carmellose sodium), hydroxypropyl cellulose (HPC) or hypromellose (hydroxypropyl methylcellulose), particularly preferably hypromellose. These binders may be used singly or in combination of two or more kinds thereof. Moreover, the additive agents described above may be used for other purposes in addition to being used as a binder.

A content of the binder in the preparation of the present invention is not particularly limited, and is 0.01 to 10.0% by weight, preferably 0.05 to 5.0% by weight, more preferably 0.1 to 2.5% by weight, based on the total amount of the preparation. In addition, the content of the binder is 1 to 30 parts by weight, preferably 2.5 to 25 parts by weight, more preferably 5 to 20 parts by weight, based on 1 part by weight of the compound used in the preparation of the present invention. When the content is out of these ranges, there is a possibility that it is difficult to produce a preparation. When two or more kinds of binders are used, the total amount of the binders may be within the above content ranges.

The preparation of the present invention may contain a lubricant, and lubricants listed in the Japanese Pharmacopoeia, the Japanese Pharmaceutical Codex, the Japanese Pharmaceutical Excipients, the Japanese Standards of Food Additives, the United States Pharmacopoeia (USP), and the European Pharmacopoeia (Ph.Eur.) can be used.

Examples of the lubricant include stearic acid and metallic stearate, an inorganic lubricant, a hydrophobic lubricant, a hydrophilic lubricant, and sodium stearyl fumarate.

Examples of the stearic acid and metallic stearate include magnesium stearate, calcium stearate, stearic acid, stearyl alcohol, and polyoxyl 40 stearate.

Examples of the inorganic lubricant include talc, light anhydrous silicic acid, hydrated silicon dioxide, magnesium carbonate, precipitated calcium carbonate, dried aluminum hydroxide gel, magnesium aluminometasilicate, magnesium silicate, synthetic aluminum silicate, magnesium oxide, and magnesium sulfate.

Examples of the hydrophobic lubricant include cacao butter, carnauba wax, glycerin fatty acid ester, hardened oil, white beeswax, soybean hardened oil, beeswax, cetanol, sodium laurate, and sodium stearyl fumarate.

Examples of the hydrophilic lubricant include sucrose fatty acid ester and polyethylene glycol (Macrogol).

The lubricant is preferably a metallic stearate or an inorganic lubricant, more preferably magnesium stearate, calcium stearate, talc, light anhydrous silicic acid, hydrated silicon dioxide, magnesium carbonate, precipitated calcium carbonate, dried aluminum hydroxide gel, magnesium aluminometasilicate, magnesium silicate, synthetic aluminum silicate, magnesium oxide or magnesium sulfate, particularly preferably magnesium stearate or talc. These lubricants may be used singly or in combination of two or more thereof. Moreover, the additive agents described above may be used for other purposes in addition to being used as a lubricant.

A content of the lubricant in the preparation of the present invention is not particularly limited, and is 0.01 to 5.0% by weight, preferably 0.02 to 2.5% by weight, more preferably 0.05 to 2.0% by weight, based on the total amount of the preparation. In addition, the content of the lubricant is 0.1 to 50 parts by weight, preferably 0.5 to 40 parts by weight, more preferably 1 to 30 parts by weight, based on 1 part by weight of the compound used in the preparation of the present invention. When the content is out of these ranges, there is a possibility that the preparation does not flow sufficiently. When two or more kinds of lubricants are used, the total amount of the lubricants may be within the above content ranges.

The preparation of the present invention may contain an anti-caking agent in order to prevent caking of the preparation and facilitate packaging, transportation, and consumption, and additive agents listed in the Japanese Pharmacopoeia, the Japanese Pharmaceutical Codex, the Japanese Pharmaceutical Excipients, the Japanese Standards of Food Additives, the United States Pharmacopoeia (USP), and the European Pharmacopoeia (Ph.Eur.) can be used as the anti-caking agent. Examples of the anti-caking agent include powder cellulose, sodium stearate, talc, silicon dioxide, colloidal silicon dioxide, calcium silicate, magnesium silicate, magnesium trisilicate, calcium trisilicate, tricalcium phosphate, and magnesium oxide. Preferred is talc. These anti-caking agents may be used singly or in combination of two or more kinds thereof. Moreover, the additive agents described above may be used for other purposes in addition to being used as an anti-caking agent.

A content of the anti-caking agent in the preparation of the present invention is not particularly limited, and is 0.1 to 5.0% by weight, preferably 0.2 to 2.5% by weight, more preferably 0.25 to 2.0% by weight, based on the total amount of the preparation. In addition, the content of the anti-caking agent is 0.1 to 50 parts by weight, preferably 0.5 to 40 parts by weight, more preferably 1 to 30 parts by weight, based on 1 part by weight of the compound used in the preparation of the present invention. When the content is out of these ranges, there is a possibility that the preparation does not flow sufficiently. When two or more kinds of anti-caking agents are used, the total amount of the anti-caking agents may be within the above content ranges.

The present preparation may contain a pigment or a color agent, pigments and color agents listed in the Japanese Pharmacopoeia, the Japanese Pharmaceutical Codex, the Japanese Pharmaceutical Excipients, the Japanese Standards of Food Additives can be used. Examples of the pigment or the color agent include ferric oxide, tar dye, and natural dye. Examples of the ferric oxide include red ferric oxide, yellow oxide of iron, yellow ferric oxide, and black oxide of iron. Examples of the tar dye include Food Yellow No. 4 aluminum lake, Food Blue No. 1 aluminum lake, Food Red No. 3 aluminum lake, Food Blue No. 1, Food Blue No. 2, Food Yellow No. 4, Food Yellow No. 5, Food Red No. 102, Food Red No. 2, and Food Red No. 3. Examples of the natural pigment include turmeric extract, β-carotine, carotine liquid, copper chlorophyllin sodium, copper chlorophyll, a rye green leaf extract powder, a rye green leaf extract dry powder, and a rye green leaf extract.

The present preparation may contain an additive agent except those mentioned above, if necessary, additive agents described listed in the Japanese Pharmacopoeia, the Japanese Pharmaceutical Codex, the Japanese Pharmaceutical Excipients, the Japanese Standards of Food Additives can be used. Moreover, the content of these additive agents may be an arbitrary ratio. Examples of the additive agent except those mentioned above include perfume, fluidity agent, and taste masking agent.

Examples of perfume include orange extract, orange oil, caramel, camphor, cinnamon bark oil, spearmint oil, strawberry extract, chocolate extract, cherry flavor, sour orange oil, pine oil, mentha oil, vanilla flavor, bitter extract, fruit flavor, peppermint extract, mixture flavor, mint flavor, menthol, lemon powder, lemon oil, and rose oil.

Examples of fluidity agent include hydrated silicon dioxide, light anhydrous silicic acid, crystal cellulose, synthetic aluminum silicate, and talc.

Examples of taste masking agent include aspartame, sucralose, glycine, sodium chloride, magnesium chloride, hydrochloric acid, dilute hydrochloric acid, citric acid and the salt, anhydrous citric acid, L-glutamic acid and the salt, succinic acid and the salt, acetic acid, tartaric acid and the salt, sodium hydrogen carbonate, fumaric acid and the salt, malic acid and the salt, glacial acetic acid, disodium inosinate, and honey.

When the additive agent is not in a range of decreasing stability of an active ingredient, an optional amount can be usually used alone or mixture.
The following embodiments are according to the invention in as far as these solid preparations contain one or more stabilizers as defined in claim 1:
As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and a stabilizer. For example, there is a solid preparation containing a compound represented by formula (IA) or its pharmaceutically acceptable salt, a stabilizer, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and ascorbic acid or its derivative. For example, there is a solid preparation containing a compound represented by formula (IA) or its pharmaceutically acceptable salt, ascorbic acid or its derivative, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sulfur-containing amino acid or its derivative. For example, there is a solid preparation containing a compound represented by formula (IA) or its pharmaceutically acceptable salt, sulfur-containing amino acid or its derivative, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sulfite or its derivative. For example, there is a solid preparation containing a compound represented by formula (IA) or its pharmaceutically acceptable salt, sulfite or its derivative, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and citric acid or its derivative. For example, there is a solid preparation containing a compound represented by formula (IA) or its pharmaceutically acceptable salt, citric acid or its derivative, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and malic acid or its derivative. For example, there is a solid preparation containing a compound represented by formula (IA) or its pharmaceutically acceptable salt, malic acid or its derivative, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and ascorbic acid. For example, there is a solid preparation containing a compound represented by formula (IA) or its pharmaceutically acceptable salt, ascorbic acid, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sodium L-ascorbate. For example, there is a solid preparation containing a compound represented by formula (IA) or its pharmaceutically acceptable salt, sodium L-ascorbate, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and erythorbic acid. For example, there is a solid preparation containing a compound represented by formula (IA) or its pharmaceutically acceptable salt, erythorbic acid, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and L-cysteine hydrochloride hydrate. For example, there is a solid preparation containing a compound represented by formula (IA) or its pharmaceutically acceptable salt, L-cysteine hydrochloride hydrate, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and L-methionine. For example, there is a solid preparation containing a compound represented by formula (IA) or its pharmaceutically acceptable salt, L-methionine, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sodium pyrosulfite. For example, there is a solid preparation containing a compound represented by formula (IA) or its pharmaceutically acceptable salt, sodium pyrosulfite, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sodium sulfite. For example, there is a solid preparation containing a compound represented by formula (IA) or its pharmaceutically acceptable salt, sodium sulfite, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, or a solvate of the compound or the salt, and anhydrous citric acid. For example, there is a solid preparation containing a compound represented by formula (IA) or its pharmaceutically acceptable salt, anhydrous citric acid, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a pharmaceutically acceptable salt of the compound represented by formula (IA), or a solvate of the compound or the salt, and DL-malic acid. For example, there is a solid preparation containing a compound represented by formula (IA) or its pharmaceutically acceptable salt, DL-malic acid, and one or more selected from the group consisting of an excipient, a disintegrant, a suspending agent, a binder, a lubricant, and an anti-caking agent.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, a stabilizer, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, ascorbic acid, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, sodium L-ascorbate, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, erythorbic acid, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, L-cysteine hydrochloride hydrate, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, L-methionine, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, sodium pyrosulfite, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, sodium sulfite, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, anhydrous citric acid, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, DL-malic acid, D-mannitol, croscarmellose sodium, hypromellose, magnesium stearate, and talc.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and a stabilizer, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and ascorbic acid or its derivative, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sulfur-containing amino acid or its derivative, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sulfite or its derivative, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and citric acid or its derivative, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and malic acid or its derivative, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and ascorbic acid, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sodium L-ascorbate, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and erythorbic acid, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and L-cysteine hydrochloride hydrate, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and L-methionine, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sodium pyrosulfite, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sodium sulfite, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) as an active ingredient, its pharmaceutically acceptable salt, and an additive agent, there is a solid preparation containing a compound represented by formula (IA) or its pharmaceutically acceptable salt, and anhydrous citric acid, and when the solid preparation is suspended in water at room temperature for 3 hours, the solid preparation is a solid preparation in which the total amount of related substances of the compound represented by formula (IA) is 4.0% or less.

As another embodiment of a solid preparation containing a compound represented by formula (IA) or its pharmaceutically acceptable salt as an active ingredient, and an additive agent, there is a solid preparation containing a compound represented by formula (IA) or its pharmaceutically acceptable salt, and DL-malic acid, and when the solid preparation is suspended in water at room temperature for 3 hours, the solid preparation is a solid preparation in which the total amount of related substances of the compound represented by formula (IA) is 4.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and a stabilizer, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and ascorbic acid or its derivative, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sulfur-containing amino acid or its derivative, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sulfite or its derivative, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and citric acid or its derivative, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and malic acid or its derivative, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and ascorbic acid, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sodium L-ascorbate, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and erythorbic acid, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and L-cysteine hydrochloride hydrate, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and L-methionine, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sodium pyrosulfite, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and sodium sulfite, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and anhydrous citric acid, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a compound represented by formula (IA) or its pharmaceutically acceptable salt as an active ingredient, and an additive agent, there is a solid preparation containing a compound represented by formula (IA) or its pharmaceutically acceptable salt, and DL-malic acid, and when the solid preparation is suspended in water at room temperature for 3 hours, the solid preparation is a solid preparation in which the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient and an additive agent, there is a solid preparation containing a p-toluenesulfonic acid salt of a compound represented by formula (IA) as an active ingredient and a stabilizer. For example, there is a solid preparation containing a p-toluenesulfonic acid salt of a compound represented by formula (IA), a stabilizer, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) as an active ingredient and ascorbic acid or its derivative. For example, there is a solid preparation containing a p-toluenesulfonic acid salt of a compound represented by formula (IA), ascorbic acid or its derivative, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) as an active ingredient and sulfur-containing amino acid or its derivative. For example, there is a solid preparation containing a p-toluenesulfonic acid salt of a compound represented by formula (IA), sulfur-containing amino acid or its derivative, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) as an active ingredient and sulfite or its derivative. For example, there is a solid preparation containing a p-toluenesulfonic acid salt of a compound represented by formula (IA), sulfite or its derivative, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and citric acid or its derivative. For example, there is a solid preparation containing a p-toluenesulfonic acid salt of a compound represented by formula (IA), citric acid or its derivative, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and malic acid or its derivative. For example, there is a solid preparation containing a p-toluenesulfonic acid salt of a compound represented by formula (IA), malic acid or its derivative, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and ascorbic acid. For example, there is a solid preparation containing a p-toluenesulfonic acid salt of a compound represented by formula (IA), ascorbic acid, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and sodium L-ascorbate. For example, there is a solid preparation containing a p-toluenesulfonic acid salt of a compound represented by formula (IA), sodium L-ascorbate, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and erythorbic acid. For example, there is a solid preparation containing a p-toluenesulfonic acid salt of a compound represented by formula (IA), erythorbic acid, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and L-cysteine hydrochloride hydrate. For example, there is a solid preparation containing a p-toluenesulfonic acid salt of a compound represented by formula (IA), L-methionine, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and L-methionine. For example, there is a solid preparation containing a p-toluenesulfonic acid salt of a compound represented by formula (IA), L-methionine, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and sodium pyrosulfite. For example, there is a solid preparation containing a p-toluenesulfonic acid salt of a compound represented by formula (IA), sodium pyrosulfite, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and sodium sulfite. For example, there is a solid preparation containing a p-toluenesulfonic acid salt of a compound represented by formula (IA), sodium sulfite, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and anhydrous citric acid. For example, there is a solid preparation containing a p-toluenesulfonic acid salt of a compound represented by formula (IA), anhydrous citric acid, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient and an additive agent, there is a solid preparation containing a p-toluenesulfonic acid salt of a compound represented by formula (IA) and DL-malic acid. For example, there is a solid preparation containing a p-toluenesulfonic acid salt of a compound represented by formula (IA), DL-malic acid, and one or more selected from the group consisting of an excipient, a disintegrant, a suspending agent, a binder, a lubricant, and an anti-caking agent.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), a stabilizer, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), ascorbic acid, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), sodium L-ascorbate, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), erythorbic acid, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), L-cysteine hydrochloride hydrate, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), L-methionine, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), sodium pyrosulfite, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), sodium sulfite, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), anhydrous citric acid, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), DL-malic acid, D-mannitol, croscarmellose sodium, hypromellose, magnesium stearate, and talc.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and a stabilizer, and when the solid preparation is suspended in water at room temperature for 3 hours, the solid preparation is a solid preparation in which the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and ascorbic acid or its derivative, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sulfur-containing amino acid or its derivative, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sulfite or its derivative, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and citric acid or its derivative, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and malic acid or its derivative, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and ascorbic acid, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sodium L-ascorbate, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and erythorbic acid, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and L-cysteine hydrochloride hydrate, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and L-methionine, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sodium pyrosulfite, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sodium sulfite, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and anhydrous citric acid, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and DL-malic acid, and when the solid preparation is suspended in water at room temperature for 3 hours, the total amount of related substances of the compound represented by formula (IA) is 4.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and a stabilizer, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and ascorbic acid or its derivative, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As another embodiment of a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sulfur-containing amino acid or its derivative, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sulfite or its derivative, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and citric acid or its derivative, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and malic acid or its derivative, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and ascorbic acid, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sodium L-ascorbate, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and erythorbic acid, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and L-cysteine hydrochloride hydrate, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and L-methionine, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sodium pyrosulfite, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and sodium sulfite, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and anhydrous citric acid, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

As a solid preparation containing a p-toluenesulfonic acid salt of a compound represented by formula (IA) as an active ingredient, and an additive agent, there is a solid preparation containing a p-toluenesulfonic acid salt of a compound represented by formula (IA), and DL-malic acid, and when the solid preparation is suspended in water at room temperature for 3 hours, the amount of a hydroxide form and/or a carboxylic acid form of the compound represented by formula (IA) among related substances of the compound represented by formula (IA) is 1.0% or less.

Dosage form of the preparation of the present invention may be any solid preparation. For example, the solid preparation may be a granule, a powder, a tablet, a fine granule, a pill or the like, and is preferably a granule or a powder. According to the Japanese Pharmacopoeia 17th edition, a granule is a granulated preparation for oral administration, and a powder is a powdery preparation for oral administration.

Among the preparations of the present invention, the method for producing a granule or a powder is not particularly limited, but the granule or powder can be produced by the following method. Examples of the method include, but are not limited to, a method of mixing the present compound, additive agents such as granular or powdery excipient and disintegrant to produce a granule or a powder, as another embodiment, a method of kneading a mixed powder obtained by mixing the present compound, and additive agents such as an excipient and a disintegrant with water or the like and then extruding and granulating the mixture, as another embodiment, a method of spraying water or the like to a mixed powder obtained by mixing the present compound, and additive agents such as an excipient and a disintegrant to perform fluidized bed granulation, as another embodiment, a method of spraying water or the like to a mixed powder obtained by mixing the present compound, and additive agents such as an excipient and a disintegrant to perform stirring granulation, as another embodiment, a method of spraying water or the like to a mixed powder obtained by mixing the present compound, and additive agents such as an excipient and a disintegrant to perform rolling granulation, as another embodiment, a method of spraying water or the like to a mixed powder obtained by mixing the present compound, and additive agents such as an excipient and a disintegrant to perform rolling fluidized bed granulation, and the like. When the granule is produced, granulation may be performed by either organic solvent except water such as ethanol, acetone, ethyl alcohol, propyl alcohol and the like, or the mixing solvent of water and organic solvent. Moreover, after producing the granules, the granules can be coated by coating agent.

The average particle diameter of the preparation of the present invention is not particularly limited, but is 1 to 1000 µm, preferably 5 to 500 µm, more preferably 10 to 250 µm, particularly preferably 100 µm in terms of volume average particle diameter.

The dose of the compound represented by formula (IA) in the preparation of the present invention is usually to be orally administered 0.01 to 1.0 mg, preferably 0.2 mg as the present compound once a day to an adult.

The preparation of the present invention may be to be administered orally directly, or may be suspended in water.

In the preparation of the present invention, no agglomerate is visually observed in the initial stage of production.

The average value of the content of the compound represented by formula (IA) in the preparation of the present invention is 95 to 105%. Also, the standard deviation of the individual contents is 4.0% or less.

Regarding dissolution of the preparation of the present invention, as a result of a test using the 2nd fluid for dissolution test in accordance with a dissolution test method in the Japanese Pharmacopoeia, the dissolution rate after 15 minutes from the start of the test is 80% or more.

The preparation of the present invention has good suspension in water. When 5 mL of water is added to about 500 mg of the preparation of the present invention, a uniform suspending agent can be visually obtained by shaking the container, particularly by shaking the container for about 15 seconds.

### [EXAMPLES]

The present invention will be explained in more detail below by way of Examples, Comparative Examples and Reference Examples, but these do not limit the present invention. The preparations of Examples, Comparative Examples and Reference Examples were produced by the following methods.

### (1) Effect of stabilizer in preparations in 2 weeks temporal stability test at 50°C

To investigate an effect of a stabilizer in preparations in a temporal stability test after storage at 50°C for 2 weeks, a preparation described in Table 1 was produced. As the stabilizer, the stabilizers of Examples 1, 7, and 9 and Comparative Examples 1 to 9 were used.

### a. Process for preparation production

A tosylate salt of the compound represented by formula (IA) was sieved through a 60 mesh sieve. D-mannitol (Pearlitol 100SD (manufactured by ROQUETTE)), croscarmellose sodium (AcDiSol, manufactured by FMC Corporation), hypromellose (TC-5, manufactured by Shin-Etsu Chemical Co., Ltd.), magnesium stearate (manufactured by Mallinckrodt Pharmaceuticals), and talc (manufactured by MERCK) were sieved through a 30 mesh sieve.

Some D-mannitol and a tosylate salt of a compound represented by formula (IA) were premixed in a polyethylene bag, and then sieved through a 30 mesh sieve. This mixed powder is hereinafter referred to as "drug substance premix powder".

On the other hand, a wire mesh and a receiving container were rinsed with some D-mannitol. The raw materials including the D-mannitol used for rinsing were premixed in a polyethylene bag.

The drug substance premix powder and the D-mannitol, croscarmellose sodium, and hypromellose were mixed with 8LV mixer. Thereafter, magnesium stearate and talc were charged and mixed with the 8LV mixer. This mixed powder is hereinafter referred to as "drug substance mixed powder".

Some of the drug substance mixed powder and a stabilizer were premixed in a plastic bag, and then sieved through a 30 mesh sieve. Thereafter, some of the drug substance mixed powder and the stabilizer were mixed in a plastic bag. In consideration of the maximum daily dose of each stabilizer, the amounts shown in Examples 1, 7, and 9 and Comparative Examples 1 to 9 in Tables 2 to 7 were blended as the stabilizers in the preparations. In addition, a preparation containing no stabilizer in the preparation was designated as Comparative Example 12, and stability was evaluated.

**[Table 1]**

| Component | Weight (mg) |
|---|---|
| Tosylate salt of Compound A | 0.2604 (free form 0.2) |
| D-Mannitol | 978.9396 |
| Croscarmellose sodium | 12.0 |
| Hypromellose | 3.2 |
| Magnesium stearate | 0.6 |
| Talc | 5.0 |
| Stabilizer | X (shown in Tables 2 to 7) |
| Total | 1000+X |

**[Table 2]**

| | Example 1 | Example 2 | Example 3 | Example 4 |
|---|---|---|---|---|
| Stabilizer | Ascorbic acid | Sodium L-ascorbate | Erythorbic acid | L-Cysteine hydrochloride hydrate |
| Contained amount (mg) | 10 | 50 | 100 | 50 |

**[Table 3]**

| | Example 5 | Example 6 | Example 7 | Example 8 |
|---|---|---|---|---|
| Stabilizer | L- Methionine | Sodium pyrosulfite | Sodium sulfite | Anhydrous citric acid |
| Contained amount (mg) | 50 | 100 | 100 | 100 |

**[Table 4]**

| | Example 9 |
|---|---|
| Stabilizer | DL-malic acid |
| Contained amount (mg) | 100 |

**[Table 5]**

| | Comparative Example 1 | Comparative Example 2 | Comparative Example 3 | Comparative Example 4 |
|---|---|---|---|---|
| Stabilizer | Riboflavin | B-Carotine | Sodium edetate hydrate | Propyl gallate |
| Contained amount (mg) | 10 | 10 | 100 | 10 |

**[Table 6]**

| | Comparative Example 5 | Comparative Example 6 | Comparative Example 7 | Comparative Example 8 |
|---|---|---|---|---|
| Stabilizer | Thymol | Soybean lecithin | Butylated hydroxyanisole | Fumaric acid |
| Contained amount (mg) | 10 | 100 | 10 | 100 |

**[Table 7]**

| | Comparative Example 9 | Comparative Example 10 | Comparative Example 11 | Comparative Example 12 |
|---|---|---|---|---|
| Stabilizer | Dibutylhydroxytoluene | α⁻Tocopherol | Tocopherol acetate | None |
| Contained amount (mg) | 10 | 50 | 50 | - |

### b. Stability test method

The preparation of the present invention was stored at 50°C for 2 weeks in a amber glass bottle closed state. A predetermined amount of the preparation of the present invention was collected, and the amount of related substances of the compound represented by formula (IA) in the sample was measured.

### (Method for preparing sample solution)

0.5 g of a sample was collected and charged into a centrifuge tube. 10 mL of a solution obtained by dissolving 1.0 g of a stabilizer in 1000 mL of a water/ methanol (3:1) mixed solution was accurately weighed and charged into the centrifuge tube containing the sample. The centrifuge tube was shaken at room temperature for 5 minutes, and then ultrasonically irradiated with occasional shaking for 2 minutes. Then, the supernatant after centrifugation (3000 rpm, 10 min) was filtered through a membrane filter with a pore size of 0.45 µm (chromatographic disk 25N, nonaqueous system) using a syringe, and 3 mL of the initial stream was discarded to obtain a sample solution.

### (Measurement method)

An amount of the compound was measured by a liquid chromatograph according to the following method and conditions.
· Detector: ultraviolet absorptiometer (measurement wavelength: 240 nm)
· Column: L-column ODS (filler 3 µm, 4.6 × 100 mm, manufactured by Chemicals Evaluation and Research Institute)
· Column temperature: constant temperature at near 45°C
· Mobile phase: 20 mmol/L phosphate buffer solution of pH 6.1/ acetonitrile for HPLC/ methanol for HPLC mixed solution (13:6:1)
· Flow rate of mobile phase: 1.0 mL/min

For the amount of related substances, the total peak area of chromatogram of HPLC chart was taken as 100%, and the ratio (%) of the amount was calculated.

### c. Result

The preparations of the present invention were stored at 50°C for 2 weeks in amber glass bottle closures. Predetermined amounts of the preparations of the present invention (Examples 1, 7 and 9 and Comparative Examples 1 to 9 and 12) were collected, and the amounts of related substances of the compound represented by formula (IA) in the sample were measured. Table 7 shows stabilizers, contained amounts of the stabilizers, maximum values (%) of amounts of individual related substances, related substances showing the maximum value, and total amounts of related substances. As a result, the total amounts of related substances were 4.0% or more and the maximum values of amounts of individual related substances were 1% or more in Comparative Examples 2, 6, and 8, but the total amounts of related substances in other Examples 1, 7, and 9 and Comparative Examples 1, 3 to 5, 7, 9, and 12 were 4% or less, and the maximum values of amounts of individual related substances in Examples 1, 7, and 9 and Comparative Examples 1, 3, 5, 7, 9, and 12 were 1% or less.

**[Table 8]**

| | Example 1 | Example 7 | Example 9 |
|---|---|---|---|
| Stabilizer | Ascorbic acid | Sodium sulfite | DL-Malic acid |
| Contained amount (mg) | 10 | 100 | 100 |
| Maximum value (%) of amounts of individual related substances | 0.07 | 0.14 | 1.16 |
| Related substance showing maximum value | Related substance other than hydroxide form and carboxylic acid form | Related substance other than hydroxide form and carboxylic acid form | Hydroxide form |
| Total amount (%) of related substances | 0.20 | 0.54 | 3.29 |

**[Table 9]**

| | Comparative Example 1 | Comparative Example 2 | Comparative Example 3 | Comparative Example 4 |
|---|---|---|---|---|
| Stabilizer | Riboflavin | B-Carotine | Sodium edetate hydrate | Propyl gallate |
| Contained amount (mg) | 10 | 10 | 100 | 10 |
| Maximum value (%) of amounts of individual related substances | 0.83 | 9.83 | 0.24 | 1.27 |
| Related substance showing maximum value | Related substance other than hydroxide form and carboxylic acid form | Carboxylic acid form | Related substance other than hydroxide form and carboxylic acid form | Related substance other than hydroxide form and carboxylic acid form |
| Total amount (%) of related substances | 1.43 | 40.04 | 0.41 | 2.58 |

**[Table 10]**

| | Comparative Example 5 | Comparative Example 6 | Comparative Example 7 | Comparative Example 8 |
|---|---|---|---|---|
| Stabilizer | Thymol | Soybean lecithin | Butylated hydroxyanisole | Fumaric acid |
| Contained amount (mg) | 10 | 100 | 10 | 100 |
| Maximum value (%) of amounts of individual related substances | 0.33 | 11.30 | 0.76 | 5.53 |
| Related substance showing maximum value | Related substance other than hydroxide form and carboxylic acid form | Related substance other than hydroxide form and carboxylic acid form | Carboxylic acid form | Related substance other than hydroxide form and carboxylic acid form |
| Total amount (%) of related substances | 1.14 | 26.46 | 2.78 | 5.59 |

**[Table 11]**

| | Comparative Example 9 | Comparative Example 12 |
|---|---|---|
| Stabilizer | Dibutylhydroxytoluene | None |
| Contained amount (mg) | 10 | - |
| Maximum value (%) of | 0.13 | 0.24 |
| amounts of individual related substances | | |
| Related substance showing maximum value | Hydroxide form | Related substance other than hydroxide form and carboxylic acid form |
| Total amount (%) of related substances | 0.59 | 0.66 |

### (2) Stability test of preparations in water

To investigate an effect of a stabilizer in water in the preparations containing the present compound, a stability test of the preparations described in Tables 2 to 7 in water was performed.

### a. Process for preparation production

A preparation described in Table 12 was produced as a preparation. Some D-mannitol (Pearlitol 100SD (manufactured by ROQUETTE)) and a tosylate salt of the compound represented by formula (IA) were premixed in a polyethylene bag, and then sieved through a 30 mesh sieve. This mixed powder is hereinafter referred to as "drug substance premix powder".

On the other hand, a wire mesh and a receiving container were rinsed with some D-mannitol. The raw materials including the D-mannitol used for rinsing were premixed in a polyethylene bag.

The drug substance premix powder and the D-mannitol, croscarmellose sodium (AcDiSol, manufactured by FMC Corporation), and hypromellose (TC-5, manufactured by Shin-Etsu Chemical Co., Ltd.) were mixed with 8LV mixer. Thereafter, magnesium stearate (manufactured by Mallinckrodt Pharmaceuticals) and talc (manufactured by MERCK) were charged, and mixed with the 8LV mixer. In consideration of the maximum daily dose of each stabilizer, the amounts shown in Tables 2 to 7 were blended as the stabilizers in the preparations.

**[Table 12]**

| Component | Weight (mg) |
|---|---|
| Tosylate salt of Compound A | 0.2604 (free form 0.2) |
| D-Mannitol | 978.9396 |
| Croscarmellose sodium | 12.0 |
| Hypromellose | 3.2 |
| Magnesium stearate | 0.6 |
| Talc | 5.0 |
| Stabilizer | X (shown in Tables 2 to 7) |
| Total | 1000+X |

### b. Stability test method in water

The preparation of the present invention may be suspended in water. Therefore, a stabilizer capable of improving stability by suspending the preparation of the present invention in tap water was studied.

### (Test method)

1.875 g of a preparation containing the tosylate of Compound A and additive agents other than the stabilizer in Table 1, and a predetermined amount of the stabilizer were put into a 100 mL container. Approximately 70 g of tap water was charged into the container and stirred. After stirring, tap water was additionally charged so that the total amount of tap water charged was 75 g to obtain a suspension. After 3 hours from the charge of tap water, stirring was performed for 30 seconds using a stirrer, a sample was collected from a central portion of the container, and the amount of related substances of the compound represented by formula (IA) in the sample was measured.

### (Method for preparing sample solution)

6 mL of the sample was collected with a whole pipette and put into a centrifuge tube. 4 mL of a solution obtained by dissolving 1.0 g of L-ascorbic acid in 400 mL of a water/ methanol (150:250) mixed solution was accurately weighed and charged into the centrifuge tube containing the sample. The centrifuge tube was shaken at room temperature for 5 minutes, and then ultrasonically irradiated with occasional shaking for 2 minutes. Then, the centrifuged (3000 rpm, 10 min) supernatant was filtered through a membrane filter with a pore size of 0.45 µm (chromatographic disk 25N, nonaqueous system) using a syringe, and 3 mL of the initial stream was discarded to obtain a sample solution.

### (Measurement method)

An amount of the compound was measured by a liquid chromatograph according to the following method and conditions.
· Detector: ultraviolet absorptiometer (measurement wavelength: 240 nm)
· Column: L-column ODS (filler 3 µm, 3.0 × 100 mm, manufactured by Chemicals Evaluation and Research Institute)
· Column temperature: constant temperature at near 45°C
· Mobile phase: 20 mmol/L phosphate buffer solution of pH 6.1/ acetonitrile for HPLC/ methanol for HPLC mixed solution (13:6:1)
· Flow rate of mobile phase: 0.4 mL/min

For the amount of related substances, the total peak area of chromatogram of HPLC chart was taken as 100%, and the ratio (%) of the amount was calculated.

### c. Result

After 3 hours from the charge of the preparation of the present invention, a sample was collected from a central portion of the container, and the amount of related substances of the compound represented by formula (IA) in the sample was measured. Tables 13 to 18 show stabilizers, maximum values (%) of amounts of individual related substances, and related substances showing the maximum value. As a result, in the preparations of Examples 1 to 9, the maximum amounts of individual related substances were all less than 1.0%, and these amounts satisfied the safety confirmation thresholds of ICH (International Conference on Harmonization) Q3B guidelines. On the other hand, in the preparations of Comparative Examples 1 to 11 and the absence of a stabilizer (Comparative Example 12), the maximum amounts of individual related substances were 1.0% or more, all of which showed high values.

Also, when the preparations of Comparative Examples 1, 3, 5, 7, 9 and 12 were stored at 50°C for 2 weeks, the maximum values of individual related substances were 1.0% or less as described above, but when these preparations were suspended in water at room temperature for 3 hours, the maximum values of individual related substances were 1.0% or more. In addition, depending on the preparation, the related substance showing the maximum value was sometimes different between when stored at 50°C for 2 weeks and when suspended in water for 3 hours. From the above, the stability when suspended in water at room temperature for 3 hours might be different from the stability when stored at 50°C for 2 weeks, and it was considered to be difficult to predict the stability in water from the temporal stability test.

**[Table 13]**

| | Example 1 | Example 2 | Example 3 | Example 4 |
|---|---|---|---|---|
| Stabilizer | Ascorbic acid | Sodium L-ascorbate | Erythorbic acid | L-Cysteine hydrochloride hydrate |
| Contained amount (mg) | 10 | 50 | 100 | 50 |
| Maximum value (%) of amounts of individual related substances | 0.57 | 0.22 | 0.13 | 0.28 |
| Related substance showing maximum value | Hydroxide form | Related substance other than hydroxide form and carboxylic acid form | Carboxylic acid form | Carboxylic acid form |

**[Table 14]**

| | Example 5 | Example 6 | Example 7 | Example 8 |
|---|---|---|---|---|
| Stabilizer | L- Methionine | Sodium pyrosulfite | Sodium sulfite | Anhydrous citric acid |
| Contained amount (mg) | 50 | 100 | 100 | 100 |
| Maximum value (%) of amounts of individual related substances | 0.63 | 0.39 | 0.94 | 0.40 |
| Related substance showing maximum value | Hydroxide form | Carboxylic acid form | Related substance other than hydroxide form and carboxylic acid form | Hydroxide form |

**[Table 15]**

| | Example 9 |
|---|---|
| Stabilizer | DL-malic acid |
| Contained amount (mg) | 100 |
| Maximum value (%) of amounts of individual related substances | 0.97 |
| Related substance showing maximum value | Carboxylic acid form |

**[Table 16]**

| | Comparative Example 1 | Comparative Example 2 | Comparative Example 3 | Comparative Example 4 |
|---|---|---|---|---|
| Stabilizer | Riboflavin | B-Carotine | Sodium edetate hydrate | Propyl gallate |
| Contained amount (mg) | 10 | 10 | 100 | 10 |
| Maximum value (%) of amounts of individual related substances | 47.04 | 13.02 | 1.15 | 19.90 |
| Related substance showing maximum value | Carboxylic acid form | Hydroxide form | Carboxylic acid form | Hydroxide form |

**[Table 17]**

| | Comparative Example 5 | Comparative Example 6 | Comparative Example 7 | Comparative Example 8 |
|---|---|---|---|---|
| Stabilizer | Thymol | Soybean lecithin | Butylated hydroxyanisole | Fumaric acid |
| Contained amount (mg) | 10 | 100 | 10 | 100 |
| Maximum value (%) of amounts of individual related substances | 61.75 | 68.21 | 62.91 | 1.41 |
| Related substance showing maximum value | Related substance other than hydroxide form and carboxylic acid form | Related substance other than hydroxide form and carboxylic acid form | Related substance other than hydroxide form and carboxylic acid form | Carboxylic acid form |

**[Table 18]**

| | Comparative Example 9 | Comparative Example 10 | Comparative Example 11 | Comparative Example 12 |
|---|---|---|---|---|
| Stabilizer | Dibutylhydroxyto luene | α-Tocopherol | Tocopherol acetate | None |
| Contained amount (mg) | 10 | 50 | 50 | - |
| Maximum value (%) of amounts of individual related substances | 3.86 | 12.29 | 1.81 | 11.10 |
| Related substance showing maximum value | Carboxylic acid form | Hydroxide form | Hydroxide form | Hydroxide form |

### (3) Stability test of preparations containing ascorbic acid and L-methionine as stabilizers

To examine stability of preparations in which ascorbic acid and L-methionine having an excellent effect as stabilizers were blended respectively, a preparation described in Table 19 was produced.

### a. Process for preparation production

Raw materials other than the drug substance were sieved through 30 mesh.

In order to produce a mixed powder of some D-mannitol and an antioxidant, the raw materials were mixed using a 2 L high speed mixer (EARTHTECHNICA Co., Ltd.) while adding water. Thereafter, the mixture was dried with dryer MP-01 (Powrex Corporation). This mixed powder is hereinafter referred to as "antioxidant mixed powder".

A tosylate salt of the compound represented by formula (IA) was sieved through a 60 mesh sieve. Some D-mannitol and a tosylate salt of a compound represented by formula (IA) were premixed in a polyethylene bag, and then sieved through a 30 mesh sieve. A wire mesh and a receiving container were rinsed with some D-mannitol. The raw materials including the D-mannitol used for rinsing were premixed in a polyethylene bag. This mixed powder is hereinafter referred to as "drug substance premix powder".

The drug substance premix powder and some D-mannitol, the antioxidant mixed powder, croscarmellose sodium, and hypromellose were mixed in a polyethylene bag. Thereafter, magnesium stearate and talc were charged into the bag and mixed.

**[Table 19]**

| Component (mg) | | |
|---|---|---|
| Component | Example 10 | Example 11 |
| Tosylate salt of Compound A | 0.2604 (free form 0.2) | 0.2604 (free form 0.2) |
| D-Mannitol (confirmation) | 980.9396 | 980.9396 |
| Croscarmellose sodium | 12.0 | 12.0 |
| Hypromellose | 3.2 | 3.2 |
| Magnesium stearate | 0.6 | 0.6 |
| Talc | 5.0 | 5.0 |
| Ascorbic acid | 10.0 | - |
| L- Methionine | - | 10.0 |
| Total | 1012.0 | 1012.0 |

### b. Stability test method

### (Test method)

The preparation of the present invention was stored at 40°C and 75% relative humidity for 1 week in a amber glass bottle open state. A predetermined amount of the preparation of the present invention was collected, and the amount of related substances of the compound represented by formula (IA) in the sample was measured.

### (Method for preparing sample solution)

0.5 g of a sample was collected and charged into a centrifuge tube. 10 mL of a solution obtained by dissolving 1.0 g of L-ascorbic acid in 1000 mL of a water/ methanol (3:1) mixed solution was accurately weighed and charged into the centrifuge tube containing the sample. The centrifuge tube was shaken at room temperature for 5 minutes, and then ultrasonically irradiated with occasional shaking for 2 minutes. Then, the supernatant after centrifugation (3000 rpm, 10 min) was filtered through a membrane filter with a pore size of 0.45 µm (chromatographic disk 25N, nonaqueous system) using a syringe, and 3 mL of the initial stream was discarded to obtain a sample solution.

### (Measurement method)

An amount of the compound was measured by a liquid chromatograph according to the following method and conditions.
· Detector: ultraviolet absorptiometer (measurement wavelength: 240 nm)
· Column: L-column ODS (filler 5 µm, 4.6 × 250 mm, manufactured by Chemicals Evaluation and Research Institute
· Column temperature: constant temperature at near 45°C
· Mobile phase: 20 mmol/L phosphate buffer solution of pH 6.1/ acetonitrile for HPLC/ methanol for HPLC mixed solution (13:6:1)
· Flow rate of mobile phase: 1.0 mL/min

The amount of related substances was calculated from the following formula.
Amount (%) of individual related substances = M_{S} × (A_{T} × F) / A_{S} × 1/200000 × 1 / 0.02604 × 100 / Mt
M_{S}: Weighing amount (mg) of reference standard of compound represented by formula (IA)
A_{T}: Peak area of individual related substances eluted after hydroxide form, other than peaks in placebo sample, among peaks obtained from sample solution
F: Sensitivity coefficient Hydroxide form; 1.3, Others; 1.0
As: Peak area of compound represented by formula (IA) obtained from standard solution
1/200000: Dilution ratio
0.02604: Display amount (mg) of compound represented by formula (IA) in 1 unit
Mt: Sampling amount (g)

### c. Result

The total amounts of related substances in the initial and after 1 week storage in the amber glass bottle opening of the preparations of the present invention at 40°C and 75% relative humidity are shown in Table 20. Here, the "initial" means before the start of the temporal stability test. As a result, the total amounts of related substances in the preparations of Examples 10 and 11 were 4.0% or less in the initial and after standing for 1 week.

**[Table 20]**

| | Example 10 | Example 11 |
|---|---|---|
| Total amount (%) of related substances in initial | 0.00 | 0.24 |
| Total amount (%) of related substances after 1 week | 0.47 | 0.53 |

### (4) Study on content of stabilizer

To examine the content of ascorbic acid in which the stability of the compound represented by formula (IA) was increased and the production of an related substances was suppressed in the preparation containing ascorbic acid as a stabilizer, preparations described in Tables 21 and 22 were produced.

### a. Process for preparation production

Additive agents other than the drug substance were sieved through 30 mesh. The drug substance and some D-mannitol were mixed in a polyethylene bag and then sieved through a 30 mesh sieve. A wire mesh and a receiving container were rinsed with some D-mannitol and then mixed in the polyethylene bag. The remaining D-mannitol and hypromellose were put into a bag and mixed in the polyethylene bag. Thereafter, magnesium stearate and talc were charged into the bag and mixed in the polyethylene bag. The mixed powder and a predetermined amount of ascorbic acid were mixed in the polyethylene bag, then sieved through a 30 mesh sieve, and mixed again.

**[Table 21]**

| | Component (mg) | | |
|---|---|---|---|
| Component | Reference Example 1 | Reference Example 2 | Reference Example 3 |
| Tosylate salt of Compound A | 0.2604 (free form 0.2) | 0.2604 (free form 0.2) | 0.2604 (free form 0.2) |
| D-Mannitol | 980.9396 | 980.9396 | 980.9396 |
| Hypromellose | 3.2 | 3.2 | 3.2 |
| Magnesium stearate | 0.6 | 0.6 | 0.6 |
| Talc | 5.0 | 5.0 | 5.0 |
| Ascorbic acid | 1.0 | 2.0 | 5.0 |
| Total | 991.0 | 992.0 | 995.0 |

**[Table 22]**

| | Component (mg) | | |
|---|---|---|---|
| Component | Example 12 | Example 13 | Example 14 |
| Tosylate salt of Compound A | 0.2604 (free form 0.2) | 0.2604 (free form 0.2) | 0.2604 (free form 0.2) |
| D-Mannitol | 980.9396 | 980.9396 | 978.9396 |
| Croscarmellose sodium | - | - | 12.0 |
| Hypromellose | 3.2 | 3.2 | 3.2 |
| Magnesium stearate | 0.6 | 0.6 | 0.6 |
| Talc | 5.0 | 5.0 | 5.0 |
| Ascorbic acid | 10.0 | 50.0 | 500.0 |
| Total | 1000.0 | 1040.0 | 1500.0 |

### b. Stability test method

### (Test method)

1.875 g of the preparation of the present invention was put into a 100 mL container. Approximately 70 g of tap water was charged into the container and stirred. After stirring, tap water was additionally charged so that the total amount of tap water charged was 75 g to obtain a suspending agent. After 3 hours from the charge of tap water, stirring was performed for 30 seconds using a stirrer, a sample was collected from a central portion of the container, and the amount of related substances of the compound represented by formula (IA) in the sample was measured.

### (Method for preparing sample solution)

6 mL of the sample was collected with a whole pipette and put into a centrifuge tube. 4 mL of a solution obtained by dissolving 1.0 g of L-ascorbic acid in 400 mL of a water/ methanol (150:250) mixed solution was accurately weighed and charged into the centrifuge tube containing the sample. The centrifuge tube was shaken at room temperature for 5 minutes, and then ultrasonically irradiated with occasional shaking for 2 minutes. Then, the centrifuged supernatant (3000 rpm, 10 min) was filtered through a membrane filter with a pore size of 0.45 µm (chromatographic disk 25N, nonaqueous system) using a syringe, and 3 mL of the initial stream was discarded to obtain a sample solution.

### (Measurement method)

An amount of the compound was measured by a liquid chromatograph according to the following method and conditions.
· Detector: ultraviolet absorptiometer (measurement wavelength: 240 nm)
· Column: L-column ODS (filler 3 µm, 3.0 × 100 mm, manufactured by Chemicals Evaluation and Research Institute)
· Column temperature: constant temperature at near 45°C
· Mobile phase: 20 mmol/L phosphate buffer solution of pH 6.1/ acetonitrile for HPLC/ methanol for HPLC mixed solution (13:6:1)
· Flow rate of mobile phase: 0.4 mL/min

### (Calculation of amount of related substances)

The amount of related substances was calculated from the following formula.
Amount (%) of individual related substances = Ms × (A_{T} × F) / As × 1/200000 × 1 / 0.02604 × 100 / Mt
Ms: Weighing amount (mg) of reference standard of compound represented by formula (IA)
A_{T}: Peak area of individual related substances eluted after hydroxide form, other than peaks in placebo sample, among peaks obtained from sample solution
F: Sensitivity coefficient Hydroxide form; 1.3, Others; 1.0
As: Peak area of compound represented by formula (IA) obtained from standard solution
1/200000: Dilution ratio
0.02604: Display amount (mg) of compound represented by formula (IA) in 1 unit
Mt: Amount of preparation (g) contained in sample collected 6 mL with whole pipette

### c. Result

After 3 hours from the charge of the preparation of the present invention, a sample was collected from a central portion of the container, and the amount of related substances of the compound represented by formula (IA) in the sample was measured. Table 23 shows the total amounts of related substances of Reference Examples 1 to 3 and Examples 12 to 14. As a result, the total amount of related substances was detected in the preparations of Reference Examples 1 to 3, but a related substance was not detected in any of the preparations of Examples 12 to 14 containing 10 mg or more of ascorbic acid in the preparation. Therefore, it became clear that the total amount of related substances decreases as the amount of ascorbic acid in the preparation increases.

**[Table 23]**

| | Reference Example 1 | Reference Example 2 | Reference Example 3 | Example 12 | Example 13 | Example 14 |
|---|---|---|---|---|---|---|
| Total amount (%) of related substances | 1.78 | 1.60 | 0.20 | 0.00 | 0.00 | 0.00 |

### [INDUSTRIAL APPLICABILITY]

By studying various stabilizers, the preparation of the present invention containing the compound represented by formula (IA) or its pharmaceutically acceptable salt improved stability in water and temporal stability. As a result, the preparation of the present invention can be suspended in water, and even children can easily take the preparation of the present invention.

## Claims

1. A solid preparation comprising a compound represented by formula (IA): 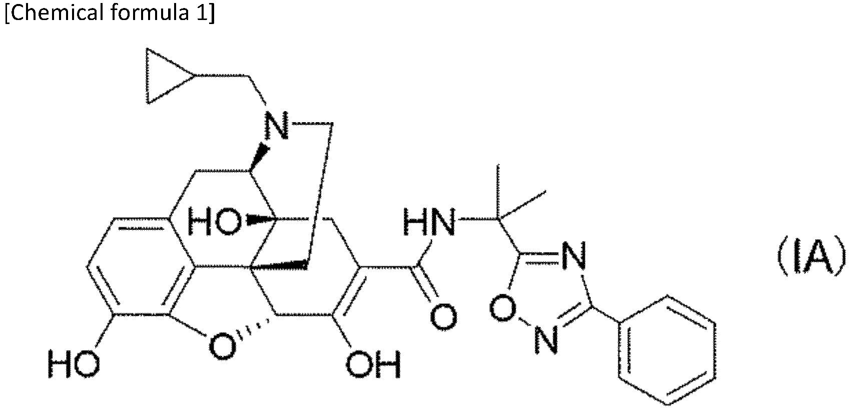 or its pharmaceutically acceptable salt as an active ingredient, and further comprising one or more stabilizers selected from the group consisting of ascorbic acid, sodium L-ascorbate, erythorbic acid, L-ascorbic acid stearic acid ester, L-ascorbic acid palmitic acid ester, L-cysteine, L-cysteine hydrochloride, L-cysteine hydrochloride hydrate, L-methionine, DL-methionine, sodium pyrosulfite, sodium sulfite, anhydrous citric acid, citric acid hydrate, calcium citrate, sodium citrate hydrate, sodium dihydrogen citrate, disodium citrate, DL-malic acid and sodium DL-malate.

2. The solid preparation according to claim 1, wherein the stabilizer is one or more selected from the group consisting of ascorbic acid, sodium L-ascorbate, erythorbic acid, L-cysteine hydrochloride hydrate, L-methionine, sodium pyrosulfite, sodium sulfite, anhydrous citric acid, and DL-malic acid.

3. The solid preparation according to claim 1, wherein the stabilizer is one or more selected from the group consisting of ascorbic acid, sodium L-ascorbate, erythorbic acid, L-cysteine hydrochloride hydrate, and L-methionine.

4. The solid preparation according to claim 1, wherein the stabilizer is ascorbic acid and/or L-methionine.

5. The solid preparation according to any one of claims 1 to 4, wherein a contained amount of the stabilizer is 1 to 2500 parts by weight, based on 1 part by weight of the compound represented by formula (IA).

6. The solid preparation according to any one of claims 1 to 5, wherein when the solid preparation is suspended in water at room temperature for 3 hours, a total amount of related substances of the compound represented by formula (IA) is 4.0% or less, wherein the related substances of the compound represented by formula (IA) comprise a hydroxide form of the compound represented by formula (IA) or a carboxylic acid form of the compound represented by formula (IA).

7. The solid preparation according to claim 6, wherein an amount of a hydroxide form and/or a carboxylic acid form of the compound represented by formula (IA) among the related substances of the compound represented by formula (IA) is 1.0% or less.

8. The solid preparation according to any one of claims 1 to 7, wherein the solid preparation is a granule or a powder.

9. The solid preparation according to claim 8, which can be suspended in water.

10. The solid preparation according to any one of claims 1 to 9, wherein the active ingredient is any of p-toluenesulfonic acid salt, acetate and hydrochloride salt of the compound represented by formula (IA).

11. A preparation obtained by suspending the solid preparation according to any one of claims 1 to 10 in a liquid.

12. The solid preparation according to any one of claims 1 to 10 or the preparation suspended in a liquid according to claim 11, which can be orally administered.

13. A method for suppressing an increase in an amount of related substances of the compound represented by formula (IA) 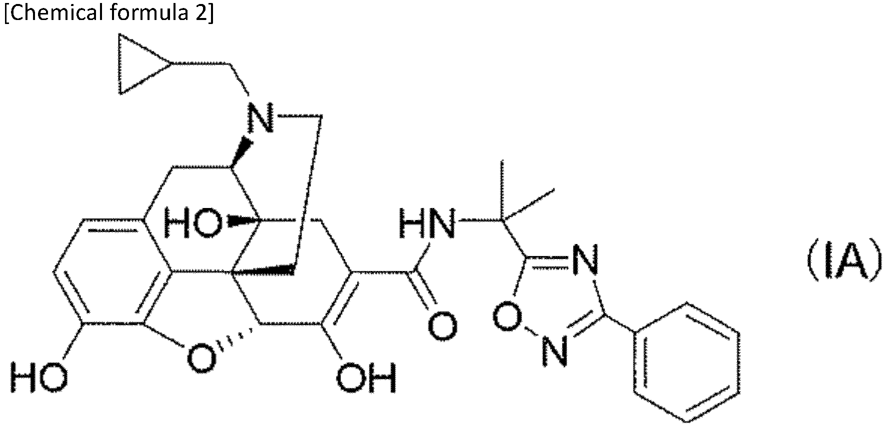 by ascorbic acid and/or L-methionine, wherein the related substances of the compound represented by formula (IA) comprise a hydroxide form of the compound represented by formula (IA) or a carboxylic acid form of the compound represented by formula (IA).

14. The method according to claim 13, which suppresses an increase in the amount of the related substances during suspension in water.

## Patentansprüche

1. Eine feste Zubereitung, umfassend eine Verbindung, dargestellt durch Formel (IA): 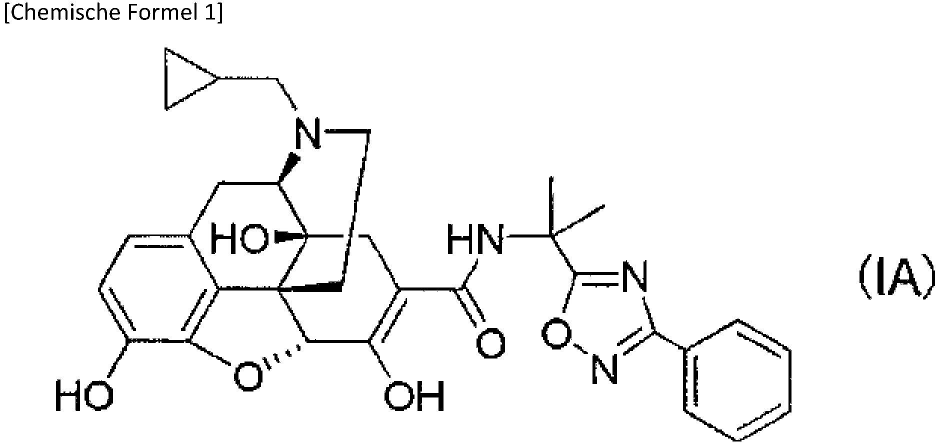 oder deren pharmazeutisch verträgliches Salz als einen Wirkstoff und weiter umfassend einen oder mehrere Stabilisatoren, ausgewählt aus der Gruppe bestehend aus Ascorbinsäure, Natrium-L-ascorbat, Erythorbinsäure, L-Ascorbinsäure-Stearinsäureester, L-Ascorbinsäure-Palmitinsäureester, L-Cystein, L-Cystein-Hydrochlorid, L-Cystein-Hydrochlorid-Hydrat, L-Methionin, DL-Methionin, Natriumpyrosulfit, Natriumsulfit, wasserfreie Zitronensäure, Zitronensäure-Hydrat, Calciumcitrat, Natriumcitrat-Hydrat, Natriumdihydrogencitrat, Dinatriumcitrat, DL-Apfelsäure und Natrium-DL-Malat.

2. Die feste Zubereitung nach Anspruch 1, wobei es sich bei dem Stabilisator um eines oder mehrere, ausgewählt aus der Gruppe bestehend aus Ascorbinsäure, Natrium-L-Ascorbat, Erythorbinsäure, L-Cysteinhydrochlorid-Hydrat, L-Methionin, Natriumpyrosulfit, Natriumsulfit, wasserfreier Zitronensäure und DL-Apfelsäure, handelt.

3. Die feste Zubereitung nach Anspruch 1, wobei es sich bei dem Stabilisator um eines oder mehrere, ausgewählt aus der Gruppe bestehend aus Ascorbinsäure, Natrium-L-Ascorbat, Erythorbinsäure, L-Cystein-Hydrochlorid-Hydrat und L-Methionin, handelt.

4. Die feste Zubereitung nach Anspruch 1, wobei der Stabilisator Ascorbinsäure und/oder L-Methionin ist.

5. Die feste Zubereitung nach einem der Ansprüche 1 bis 4, wobei eine enthaltene Menge des Stabilisators 1 bis 2500 Gewichtsteile, bezogen auf 1 Gewichtsteil der durch Formel (IA) dargestellten Verbindung, beträgt.

6. Die feste Zubereitung nach einem der Ansprüche 1 bis 5, wobei, wenn die feste Zubereitung für 3 Stunden bei Raumtemperatur in Wasser suspendiert ist, eine Gesamtmenge an verwandten Substanzen der durch Formel (IA) dargestellten Verbindung 4,0% oder weniger beträgt, wobei die verwandten Substanzen der durch Formel (IA) dargestellten Verbindung eine Hydroxidform der durch Formel (IA) dargestellten Verbindung oder eine Carbonsäureform der durch Formel (IA) dargestellten Verbindung umfassen.

7. Die feste Zubereitung nach Anspruch 6, wobei eine Menge einer Hydroxidform und/oder einer Carbonsäureform der durch Formel (IA) dargestellten Verbindung unter den verwandten Substanzen der durch Formel (IA) dargestellten Verbindung 1,0% oder weniger beträgt.

8. Die feste Zubereitung nach einem der Ansprüche 1 bis 7, wobei die feste Zubereitung ein Granulat oder ein Pulver ist.

9. Die feste Zubereitung nach Anspruch 8, welche in Wasser suspendiert sein kann.

10. Die feste Zubereitung nach einem der Ansprüche 1 bis 9, wobei der Wirkstoff einer aus p-Toluolsulfonsäuresalz, Acetat und Hydrochloridsalz der durch Formel (IA) dargestellten Verbindung ist.

11. Eine Zubereitung, erhalten durch Suspendieren der festen Zubereitung nach einem der Ansprüche 1 bis 10 in einer Flüssigkeit.

12. Die feste Zubereitung nach einem der Ansprüche 1 bis 10 oder die in einer Flüssigkeit suspendierte Zubereitung nach Anspruch 11, welche oral verabreicht werden können.

13. Ein Verfahren zur Unterdrückung eines Anstiegs einer Menge an verwandten Substanzen der durch Formel (IA) dargestellten Verbindung 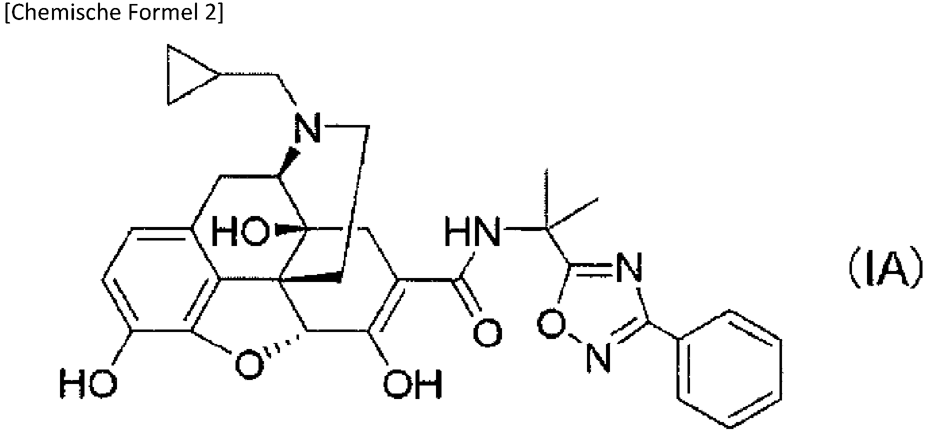 durch Ascorbinsäure und/oder L-Methionin, wobei die verwandten Substanzen der durch Formel (IA) dargestellten Verbindung eine Hydroxidform der durch Formel (IA) dargestellten Verbindung oder eine Carbonsäureform der durch Formel (IA) dargestellten Verbindung umfassen.

14. Das Verfahren nach Anspruch 13, welches einen Anstieg der Menge der verwandten Substanzen während der Suspension in Wasser unterdrückt.

## Revendications

1. Préparation solide comprenant un composé représenté par la formule (IA) : 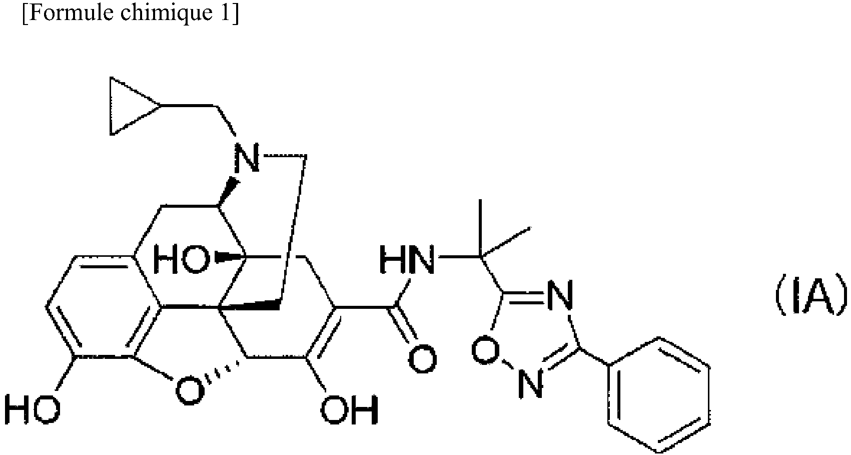 ou un son sel pharmaceutiquement acceptable en tant que principe actif, et comprenant en outre un ou plusieurs stabilisants choisis dans le groupe constitué par l'acide ascorbique, le L-ascorbate de sodium, l'acide érythorbique, l'ester d'acide L-ascorbique et d'acide stéarique, l'ester d'acide L-ascorbique et d'acide palmitique, la L-cystéine, le chlorhydrate de L-cystéine, un hydrate de chlorhydrate de L-cystéine, la L-méthionine, la DL-méthionine, le pyrosulfite de sodium, le sulfite de sodium, l'acide citrique anhydre, un hydrate d'acide citrique, le citrate de calcium, un hydrate de citrate de sodium, le dihydrogénocitrate de sodium, le citrate disodique, l'acide DL-malique et le DL-malate de sodium.

2. Préparation solide selon la revendication 1, dans laquelle le stabilisant est un ou plusieurs éléments choisis dans le groupe constitué par l'acide ascorbique, le L-ascorbate de sodium, l'acide érythorbique, l'hydrate de chlorhydrate de L-cystéine, la L-méthionine, le pyrosulfite de sodium, le sulfite de sodium, l'acide citrique anhydre, et l'acide DL-malique.

3. Préparation solide selon la revendication 1, dans laquelle le stabilisant est un ou plusieurs éléments choisis dans le groupe constitué par l'acide ascorbique, le L-ascorbate de sodium, l'acide érythorbique, l'hydrate de chlorhydrate de L-cystéine, et la L-méthionine.

4. Préparation solide selon la revendication 1, dans laquelle le stabilisant est l'acide ascorbique et/ou la L-méthionine.

5. Préparation solide selon l'une quelconque des revendications 1 à 4, dans laquelle une quantité contenue du stabilisant est de 1 à 2500 parties en poids, sur la base de 1 partie en poids du composé représenté par la formule (IA).

6. Préparation solide selon l'une quelconque des revendications 1 à 5, dans laquelle, lorsque la préparation solide est suspendue dans de l'eau à température ambiante pendant 3 heures, une quantité totale des substances apparentées du composé représenté par la formule (IA) est de 4,0 % ou moins, dans laquelle les substances apparentées du composé représenté par la formule (IA) comprennent une forme hydroxyde du composé représenté par la formule (IA) ou une forme acide carboxylique du composé représenté par la formule (IA).

7. Préparation solide selon la revendication 6, dans laquelle la quantité de la forme hydroxyde et/ou de la forme acide carboxylique du composé représenté par la formule (IA) parmi les substances apparentées du composé représenté par la formule (IA) est de 1,0 % ou moins.

8. Préparation solide selon l'une quelconque des revendications 1 à 7, dans laquelle la préparation solide est un granulé ou une poudre.

9. Préparation solide selon la revendication 8, qui peut être suspendue dans de l'eau.

10. Préparation solide selon l'une quelconque des revendications 1 à 9, dans laquelle le principe actif est l'un quelconque parmi un sel d'acide p-toluènesulfonique et le sel chlorhydrate du composé représenté par la formule (IA).

11. Préparation obtenue en suspendant la préparation solide selon l'une quelconque des revendications 1 à 10 dans un liquide.

12. Préparation solide selon l'une quelconque des revendications 1 à 10 ou préparation suspendue dans un liquide selon la revendication 11, qui peut être administrée par voie orale.

13. Méthode pour supprimer une augmentation dans une quantité de substances apparentées du composé représenté par la formule (IA) 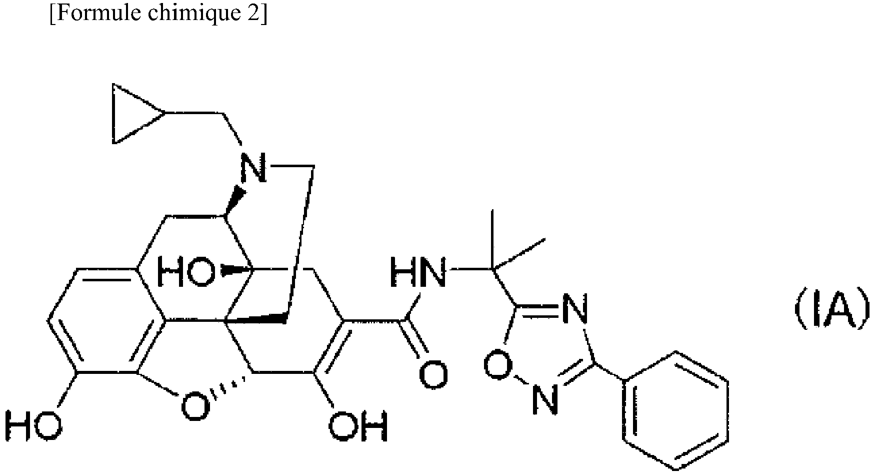 par l'acide ascorbique et/ou la L-méthionine, dans laquelle les substances apparentées du composé représenté par la formule (IA) comprennent la forme hydroxyde du composé représenté par la formule (IA) ou la forme acide carboxylique du composé représenté par la formule (IA).

14. Méthode selon la revendication 13, qui supprime l'augmentation de la quantité des substances apparentées pendant la suspension dans de l'eau.
